(19) Europäisches Patentamt
European Patent Office
Office européen des brevets

(11) Publication number: **0 443 027 B1**

(12) # EUROPEAN PATENT SPECIFICATION

(45) Date of publication of patent specification: **14.07.93 Bulletin 93/28**

(51) Int. Cl.$^5$: **A61K 47/36**

(21) Application number: **89912499.4**

(22) Date of filing: **10.11.89**

(86) International application number: **PCT/JP89/01152**

(87) International publication number: **WO 90/04980 17.05.90 Gazette 90/11**

(54) **DRUG COMPOSITION.**

(30) Priority: **11.11.88 JP 285566/88**

(43) Date of publication of application: **28.08.91 Bulletin 91/35**

(45) Publication of the grant of the patent: **14.07.93 Bulletin 93/28**

(84) Designated Contracting States: **CH DE FR GB LI**

(56) References cited:
**DE-A- 3 527 482**
**CHEM. PHARM. BULL., vol. 38, no. 1, January 1990, pages 185-187; S. SHIRAISHI et al.: "Enhancement of dissolution rates of several drugs by low-molecular chitosan and alginate"**
**CHEM. PHARM. BULL., vol. 30, no. 12, 1982, pages 4464-4467; Y. SAWAYANAGI et al.: "Enhancement of dissolution properties of griseofulvin from ground mixtures with chitin or chitosan"**

(56) References cited:
**J.P. ZIKAKIS: "Chitin, Chitosan, and Related Enzymes", PROCEEDINGS OF THE JOINT US-JAPAN SEMINAR ON ADVANCES IN CHITIN, CHITOSAN, AND RELATED ENZYMES, Delaware, Newark, 24th - 27th April 1984, Academic Press, London, GB**

(73) Proprietor: **KURITA WATER INDUSTRIES LTD.
4-7, Nishishinjuku 3-chome
Shinjuku-ku Tokyo 160 (JP)**

(72) Inventor: **HASHIMOTO, Masanori Kurita Water Industries, Ltd.
4-7, Nishishinjuku 3-chome
Shinjuku-ku Tokyo 160 (JP)**
Inventor: **OTAGIRI, Masaki
1675-32, Nagamine-machi Kumamoto-shi
Kumamoto 862 (JP)**
Inventor: **IMAI, Teruko
255, Hanatate 2-chome Kumamoto-shi
Kumamoto 861-21 (JP)**

(74) Representative: **Harrison, Ivor Stanley et al
Withers & Rogers 4 Dyer's Buildings Holborn
London EC1N 2JT (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a drug composition containing a chitosan having an average molecular weight of between 500 and 50 x $10^3$ and one or more drugs being poorly water soluble and thereby having poor or insufficient bioavailability.

Medicines called poorly water-soluble drugs have very poor solubility in water and, thus, exhibit a quite low "bioabsorptivity", namely, rate of absorption of drug in living organisms upon oral administration, often accompanied by a poor "biouptake", namely, amount of drug held absorbed in the body of living organisms. Therefore, attempts have been proposed for making such poorly water-soluble drugs easily soluble in water by converting them into water-soluble salts, e.g. sodium salt or hydrochloride salt, prescribing the drug as a water-soluble prodrug and so on. Proposals have been made also from the pharmaceutical point of view, for example, addition of a solubilizing agent, such as a surface active agent, to the drug, enclusion of the drug particles with, such as, cyclodextrin. In a fine crystallographic technology, a micropulverization of the original drug powder and treatment for making the drug powder amorphous were also proposed.

Since however, each of these prior art measures has a preferential group of drugs to apply to, no practical measure generally applicable for every drug preparation has yet been proposed. It is the stand of the technique today, that, in particular, a prior art proposal capable of complying with the demand of improvement of the biouptake of drug may not pertain to the improvement of the bioadsorptivity of drug. Among the poorly water-soluble drugs however, there are many which are requested to be furnished with an immediate medicinal effect, so that a demand still remains for realizing pharmaceuticals for oral administration which are desirable not only in the larger biouptake but also in the prompt bioabsorptivity.

In these circumstances, it has been studied to utilize various high molecular weight substances for improving the bioabsorptivity of poorly water-soluble drugs.

As concerns the use of high molecular weight gelatine, the Japanese Patent Application Kokai No. 26615/1982 discloses a technique of co-crushing of poorly water-soluble drugs with high molecular weight gelatine etc. This technique employs a large amount of gelatine to be added for improving the bioabsorptivity of poorly water-soluble drug and has a shortcoming that the technique is limited only to the employment of co-crushing.

On the other hand, attempts have been casted in the use of chitin or chitosan obtained from the shell of crab and lobster in pharmacy. Most of the uses of chitin and chitosan have, however, been aimed only at a gradual liberation of the pharmaceutical in the living organisms and there is yet only a few reports concerning solubilization of poorly water-soluble drug.

Japanese Patent Publication No. 28414/1988 discloses a method for improving the bioabsorptivity and the biouptake of either one of poorly water-soluble drugs selected from the group consisting of antibiotics and antiepileptics, by subjecting the drug to mixing and co-crushing together with chitin and/or chitosan up to such an extent that most of the drug would have been rendered amorphous. This Japanese Patent Publication does disclose, however, no use of low molecular weight chitosan and the method exhibits problems not only in the requirement of large energy consumption and extended time for the mixing and co-crushing but also in the complex pharmaceutical process.

There is, on the other hand, a report as to a technical measure for improving the dissolution rate of kitasamycin by mixing it with various high molecular weight substances on a roller mixer [ "Hyomen" 26 (5), 336 (1988)] , in which chitosan is employed in a form of a solution of acetic acid salt, of which effect is lower than that of polyvinylpyrrolidone as seen by comparison of the data.

An object of the present invention is to solve the problems explained above and to provide a drug composition in which the solubility and the rate of dissolution of various poorly water-soluble drugs are improved.

Another object of the present invention is to provide a drug composition containing one or more poorly water-soluble drugs which composition is capable of being prepared in various forms by means of various pharmacological processes in wet and dry systems, co-crushing, spray granulation and so on.

## DISCLOSURE OF THE INVENTION

The inventors had been on study for evaluating the effect of addition of various high molecular weight substances to drug compositions under the purpose of improving the dissolution performance of poorly water-soluble medicinal drugs and found thereby that the solubility and the dissolution rate of poorly water-soluble drugs were able to be improved by mixing the poorly water-soluble drugs with a chitosan, having an average molecular weight between 500 and $50 \cdot 10^3$ wherefrom the present invention has been completed.

Thus, the present invention relates to a drug composition containing one or more poorly water-soluble drugs and a low molecular weight chitosan.

The low molecular weight chitosan to be used in the drug composition according to the present invention is a molecular weight degraded product of chitosan derived from natural chitin, which is obtained by subjecting such chitosan to a molecular weight-degrading treatment and which has a weight average molecular weight in the range from 500 to 50× $10^3$, preferably in the range from 800 to 10× $10^3$, and is soluble not only in acidic water but also in neutral or alkaline water. If the weight average molecular weight is lower than 500 or greater than 50× $10^3$, the effect of improvement in the solubility and in the dissolution rate of the poorly water-soluble drug becomes poor. The weight average molecular weight can be determined by a gel permeation chromatography using a series of polyethylene glycols of known molecular weights as the calibration standards.

The starting chitosan to be employed as the raw stock for producing the low molecular weight chitosan according to the present invention is a high molecular weight substance obtained from the chitin obtained from shell or exoskeleton of crabs and lobsters by a deacetylation treatment thereof with alkali. The degree of deacetylation is not critical and is not specifically limited so long as the resulting raw chitosan can be dissolved in acidic water. The degree of deacetylation may, in general, be 50 - 100 %. The low molecular weight chitosan can be obtained from such raw chitosan by various chemical ways including enzyme treatment of the raw chitosan, treatment of the raw chitosan with, such as, hydrogen peroxide, nitrite ion, an alkali or an acid, to break down the glucoside linkages.

Practical procedure for obtaining the low molecular weight chitosan in chemical way comprises suspending the raw chitosan in an alkaline solution, adding to the resulting suspension an adequate amount of hydrogen peroxide, effecting the reaction at a definite temperature for a definite period of time to lower the molecular weight and subjecting the resulting reaction product to a refining process consisting of desalting, molecular weight fractionation and so on, followed by dewatering, drying and pulverization of the purified product. The reaction of the raw chitosan with hydrogen peroxide may preferably be carried out under the condition of a pH of 6 - 12, a concentration of hydrogen peroxide of 0.005 - 10 % by weight, a reaction temperature of 20 - 90 °C and a reaction duration of 30 - 500 minutes.

On an enzyme treatment, the raw chitosan is contacted with a chitinase or with a chitosanase to cause degradation of the molecular weight, whereupon the after-treatment as explained above may be employed.

As the poorly water-soluble drug to be contained in the drug composition according to the present invention, every medicament may be employed without special limitation so long as it exhibits poor or insufficient bioavailability, i.e. bioabsorptivity and biouptake. Examples of such medicaments include:

1) Hypnotics and sedatives, such as,
nitrazepam, triazolam, phenobarbital, amobarbital
2) Antiepileptics, such as,
phenytoin, metharbital, primidone, clonazepam, carbamazepine, valproates
3) Analgesic-antipyretic antiphlogistics, such as,
flurbiprofen, mefenamic acid, ketoprofen, ibuprofen, indomethacin, diclofenac acid, phenacetin, oxyphenbutazone, phenylbutazone, sulpyrine, pentazocine, piroxicam
4) Antiemetics, such as,
meclizine hydrochloride, dimenhydrinate
5) Psycholeptics, such as,
haloperidol, meprobamate, chlordiazepoxide, diazepam, oxazepam, sulpiride
6) Antispasmodics, such as,
papaverine, atropine, etomidoline
7) Cardiotonics, such as,
digoxin, digitoxin, methyldigoxin, ubidecarenone
8) Antiarrhythmic drugs, such as,
pindolol, ajmaline, disopyramide
9) Diuretics, such as,
hydrochlorothiazide, spironolactone, triamterene, furosemide, bumetanide
10) Antihypertensive drugs, such as,
reserpine, dihydroergotoxine mesylate, prazosin hydrochloride, metoprolol, propranolol, atenolol
11) Coronary vasodilators, such as,
nitroglycerin, isosorbide dinitrate, diltiazem, nifedipine, dipyridamole
12) Antitussives, such as,
noscapine, salbutamol, procaterol, tulobuterol, tranilast, ketotifen
13) Cerebral circulation improving drugs, such as,
nicardipine, vinpocetine
14) Antibiotics, such as,
erythromycin, josamycins, chloramphenicol, tetracycline, rifampicin, griseofulvin

15) Antihistaminics, such as,
diphenhydramine, promethazine, mequitazine
16) Steroids, such as,
triamcinolone, dexamethasone, betamethasone, prednisolone, danazol, methyltestosterone, chlormadinone acetate
17) Vitamins, such as,
vitamin E, vitamin K, $\alpha$-calcidol, phytonadione, dl-$\alpha$-tocopherol nicotinate, menatetrenone
18) Others including
dicumarol, cinnarizine, clofibrate, gefarnate, cimetidine, probenecid, mercaptopurine, methotrexate, ursodeoxycholic acid, dihydroergotamine mesylate

In view of the dissolution performance of the intrisic powdery drug, the original raw stock obtained after crushing on a wet or dry crusher may preferably have an average particle size below 100 μm, preferably below 50 μm.

The drug composition according to the present invention is obtained by mixing the low molecular weight chitosan with one or more poorly water-soluble drugs. It is desirable upon the mixing to attain a uniform dispersion of the low molecular weight chitosan over the entire pulverous mass of the poorly water-soluble drug. There is no special restriction as to the practical manner of such mixing. Thus, there may be incorporated, for example, simple mixing of the two essential powdery components (hereinafter, the mixture resulting from such simple mixing is expressed as "physical mixture"), mixing under kneading of the two essential powdery components with addition of an adequate amount of a solvent, such as water, to the mixture (hereinafter, the mixture resulting from such kneading process is denoted as "solid dispersion") and admixing of powder of the poorly water-soluble drug into an aqueous solution of the low molecular weight chitosan. An adequate amount of the low molecular weight chitosan may be employed on the mixing, in accordance with each specific drug to be mixed. In general, from 0.2 to 10 parts by weight of the low molecular weight chitosan are employed per one part of the poorly water-soluble drug for attaining an improvement in the solubility and in the dissolution rate of the drug.

The drug composition according to the present invention can be administered to human after it has been prepared into a medicinal drug in various ways. Thus, the drug composition according to the present invention can be applied as such in the form of granule or in a form of, such as, tablet, capsule, paste, patch coating, suppository, syrup, and troche. These preparations may contain, if necessary, various additives known in the pharmaceutical technique, such as, excipients, crumbling adjuvants, and lubricating agent.

The drug composition according to the present invetion can be worked up into various pharmaceutical preparations by the following procedures:

Tablet can be obtained, for example, on a wet process, by a kneading technique, in which the low molecular weight chitosan and the poorly water-soluble drug are kneaded using a suitable solvent, such as, water, an acid solution or so on, followed by drying, grain size sorting and tabletting, or by a semidirect tabletting technique, in which the low molecular weight chitosan and the poorly water-soluble drug are kneaded, followed by drying, grain size sorting, addition of various additives and tabletting; on a dry process or on a co-crushing technique, in which the amount of the low molecular weight chitosan can be adjusted. Among them, the wet process for preparing solid dispersion is at the most preferred. While it is preferable also for the granule product and capsule product to effect the pharmacological preparation on a wet process, as in the case of tablet, a dry process and the co-crushing technique, and even a spray granulation technique, may occasionally be employed. In the case of tablet and granule, coating can be incorporated for the purpose of masking of drug particle.

The drug products prepared as above exhibit an improved solubility and an improved rate of dissolution as compared with those of prior ones, so that they are superior in the bioavailability.

According to the present invention, drug compositions exhibiting improved solubility and improved rate of dissolution of the poorly water-soluble drug as compared with those of conventional ones can be obtained, by incorporation of a low molecular weight chitosan in the drug composition. These drug compositions can be worked up into various application forms by various drug preparation technique in wet and dry processes, as well as co-crushing technique, spray granulation technique and so on. The medicinal preparations made from the drug composition according to the present invention offer an improvement in the bioabsorptivity and biouptake by living organisms.

## BRIEF EXPLANATION OF THE DRAWINGS

Fig. 1 is a graph representing the experimental results of Example 1.
Figs. 2 and 3 are graphs representing the experimental results of Example 2.

Figs. 4(A) - (L) are graphical recitation of the experimantal results of Example 3.
Figs. 5(A) - (G) are graphical recitation of the experimantal results of Example 4.
Figs. 6(A) - (D) are graphical recitation of the experimantal results of Example 5.
Figs. 7(A) and 7(B) are graphs showing the results of Example 6.
Fig. 8 is a graph showing the results of Example 8.

BEST MODES FOR CARRYING OUT THE INVENTION

Below, the present invention will be described by way of Examples, in which % values are on the weight basis.

Reference Example 1

A crude pulverous chitosan (referred to hereinafter as C-0) was obtained from a crab Reptantia chionoecetes in a usual technique. From this crude pulverous chitosan, an aqueous suspension having a pH of 11.0 was prepared. To this suspension was added a varying amount of aqueous hydrogen peroxide solution each in an amount corresponding to each definite amount of hydrogen peroxide within the range of 2.1 - 40.0 g/l and the reaction was carried out while agitating the suspension at a temperature of 70 °C for 110 - 300 minutes to effect a molecular weight degradation. After desalting, purifying and freeze drying the resulting reaction product, four low molecular weight chitosan products C-1, C-2, C-3 and C-4 were obtained. On the other hand, an amount of the crude chitosan C-0 was subjected to a similar reaction with hydrogen peroxide under a condition of a pH of 8.0, a temperature of 70 °C and an initial concentration of hydrogen peroxide of 40 g/l, with agitation to effect a molecular weight degradation. After the reaction, the reaction mixture was filtered to remove solids and the filtrate was subjected to a molecular weight fractionation using a UF membrane. The resulting product was freeze dried to obtain a low molecular weight chitosan product C-5. The material properties of each of the resulting chitosan products are recited in Table 1.

The value for the evaporated residue in Table 1 is the amount of dry residue left after evapolation of an aqueous solution of the chitosan product at 105°C. The value for the ash in Table 1 corresponds to the amount of ignition residue of the chitosan product left after burning thereof at 600°C. The molecular weight of the chitosan product was determined by a gel permeation chromatography. Here, an amount of chitosan product is dissolved in an adequate amount of water with acetic acid in an amount corresponding to that of the chitosan product, from which each 2.0 ml were pipetted and were diluted using a 0.2 M acetic acid-0.1M sodium acetate buffer solution up to 50 ml and this was introduced into a column packed with TSK Gel 3000 PWXL (trade mark) of Toso K.K. and the column was eluted with a 0.2 M acetic acid-0.1 M sodium acetate buffer solution. Besides, a calibration curve for the packed column was prepared beforehand using a series of polyethylene glycol products of known molecular weights. The weight average molecular weight of each of the chitosan products was determined basing upon this calibration curve.

Table 1

| Property | Chitosan Product | | | | | |
|---|---|---|---|---|---|---|
| | C-0 | C-1 | C-2 | C-3 | C-4 | C-5 |
| Evapor. residue 1) | 92.1 | 93.7 | 93.9 | 94.2 | 98.0 | 94.0 |
| Ash 2) | 0.7 | 0.1 | 0.2 | 0.1 | 0.2 | 2.3 |
| Molecular weight 3) | 230 | 48 | 33 | 25 | 10 | 3.8 |
| Charge density 4) | 5.1 | 5.5 | 5.5 | 5.5 | 5.4 | 2.8 |

Notes: 1) Wt. %, based on the sample weight
2) Wt. %, based on the sample weight
3) × $10^3$
4) Ionic charge in meq per gram chitosan

Example 1

Each 3 parts by weight of the chitosan products C-0 to C-5 was put together with 1 part by weight of of flurbiprofen (denoted sometimes hereinafter as FP) and the mixture was kneaded with addition of water in an amount of 2-fold of that of chitosan product for 1 hour. The kneaded mixture was dried under a reduced pressure at room temperature for 48 hours. A fraction of particles passed through 100-mesh standard sieve was employed as the solid dispersion. For each of these solid dispersions, the dissolution rate of FP into water at 37 °C was observed by the procedure given below:

500 ml of water were charged in a dissolution rate measuring device (made by Toyama Sangyo Co., Ltd.) maintained at 37°C and thereto was further added each of the above solid dispersions each in an amount corresponding to 40 mg as FP and the mixture was agitated at 91 rpm. The so agitating mixture was sampled each in an amount of 3 ml at a regular time interval with a pipette fitted with a cotton plug. Each sample was filtered through a 0.45 μm membrane filter and the FP concentration of the filtrate was determined after extraction with chloroform. Results are given in Fig. 1 as a graph.

Example 2

A composition composed of vitamin E or vitamin K with the low molecular weight chitosan C-5 was prepared in the manner given below and the solubility of vitamin E or of vitamin K was determined in the manner given below.

Each 5 mg of vitamin E and vitamin K were weighed accurately (this amount is chosen so as to exceed above the solubility limits of these drugs) and were placed each in a test tube. To each of the test tubes, an equal amount of an aqueous solution of the chitosan product C-5 as obtained in Reference Example 1 of varying concentration was added and the test tube was tightly sealed and was shaken at 25 °C for 10 days. Each of the solutions which have reached to the dissolution equilibrium was sampled with a pippette fitted with a cotton plug. The sample was filtered through a 0.45 μm membrane filter and the concentration of vitamin E or vitamin K was determined by means of UV-determination after extraction with chloroform.

The experimental results for vitamin E are given in Fig. 2 and that for vitamin K in Fig. 3 each as a graph.

Example 3

Each of the drug compositions of poorly water-soluble drugs given in Table 2 composed with the low molecular weight chitosan product C-5 was examined for the solubility of the drug in the same manner as in Example 2. The results are summarily given in Figs. 4(A) to 4(L) each in a respective graph.

Table 2

| Drug | Main Application |
|---|---|
| Betamethasone | Antiinflammatory |
| Flurbiprofen | Analgesic antiinflammatory |
| Mefenamic acid | Antiinflammatory analgesic |
| Prednisolone | Glucocorticoid, Antirheumatic and Antiinflammatory |
| Ketoprofen | Analgesic antiinflammatry |
| Digoxin | for Pulmonary edema and Congestive cardiac insufficiency |
| Triamcinolone | Adrenocorticotropic hormones, Antirheumatic, Antiallergic |
| Ibuprofen | Antifebrile antiinflammatory analgesic |
| Dicumarol | Antiblood-coagulant |
| Indomethacin | Antiinflammatory analgesic antifebrile |
| Diclofenac acid | Analgesic antiinflammatory |
| Phenytoin | Antiepileptic |

As seen from Fig. 4, the solubility of each drug was found to be increased in accordance with the increase in the concentration of the low molecular weight chitosan. A marked increase in the solubility was observed, in particular, for acidic drugs. This may be due to a participation of some interaction, such as, formation of counter ions between the low molecular weight chitosan which is a basic polysaccharide and the acidic drug. However, an increase in the solubility of the drug was recognized also for steroids, namely, digoxin and phenytoin, for which a consideration for dissociation of the drug may be unnecessary. A dissolution characteristic curve of Bs-type including a rising portion of increasing solubility, a plateau and a falling portion was observed for betamethasone and triamcinolone. These results suggest that the solubility of drug is increased not only by the formation of counter ions between the low molecular weight chitosan and the drug but also by some other interaction possibly occurring between them.

Example 4

Drug dissolution rate was examined for each of the solid dispersions and for drug itself for triamcinolone, batamethasone, prednisolone, flurbiprofen, indomethacin, digoxin and phenytoin.

The rate of dissolution was determined for drug compositions having the low molecular weight chitosan C-5 obtained in Reference Example 1, by observing the concentration of the drug in the same manner as in Example 1 at a regular time interval except that the drug was charged in an amount within the range from 10 to 30 mg in 60 ml of solution and the agitation was effected at 57 rpm. The ratio of the dissolved amount of drug to the charged total amount of the drug was calculated. Results are summarized in Figs. 5(A) - 5(G) each in a corresponding graph. The experimental results for flurbiprofen are given here again.

The average dissolution time calculated from the rate of dissolution for each drug is recited in Table 3. The calculation of the average dissolution time was made basing on the method given in "Chem. Pharm. Bull.", 30, 1088 (1982).

Table 3

| Drug | Average Dissolution Time (min.) | |
|---|---|---|
| | Drug alone | Solid Dispersion |
| Triamcinolone | 24.3 | 2.7 |
| Betamethasone | 45.9 | 1.0 |
| Prednisolone | 18.7 | 1.6 |
| Flurbiprofen | 46.1 | 5.2 |
| Indomethacin | 24.8 | 16.2 |
| Digoxin | 40.9 | 3.8 |
| Phenytoin | 35.9 | 6.4 |

For all the drugs examined, the dissolution rate of the drug from the solid dispersion thereof was found to be markedly accelerated as compared with that for the drug without any additive. While there may be possible increase in the dissolution rate due to the increase in the solubility of the drug for acidic drugs as seen from the results of Example 3, it is to be pointed out that a marked increase in the dissolution rate for drugs for which the increase in the solubility was not much recognized was observed.

Example 5

A physical mixture obtained by simply mixing flurbiprofen with the low molecular weight powder product C-5 as obtained in Reference Example 1 in a weight ratio of 1 : 3 and a solid dispersion of flurbiprofen with the product C-5 prepared in the same manner as in Example 1 except that the weight mixing proportion was settled to be 1 : 3 were examined by powder X-ray diffraction. The powder X-ray diffraction examination was carried out by employing an X-ray diffraction apparatus Geiger Flex 2102 of the firm Rigakudenki K.K. with Ni filter using Cu-Ka ray under the condition of 30 kV, 20 mA, a time constant of 2 sec. and a scanning velocity of 1 °C /min.

Similar powder X-ray diffraction examination was carried out also for indomethacin, digoxin and triamcinolone in the same manner as above. The results are recited in Figs. 6(A) - 6(D) each in an X-ray diffraction chart.

The powder X-ray diffraction pattern provides informations of the drug about its crystalline structure and its state of cohesion. As seen from Figs. 6(A) - 6(D), characteristic peaks specific for each drug were observed for the physical mixture, since the low molecular weight chitosan is an amorphous substance. For the solid dispersion of acidic drug, namely, flurbiprofen, the characteristic peaks specific for the drug which were observed for the physical mixture were not detected, indicating that flurbiprofen is present therein in a state finely dispersed within the amorphous mass of the low molecular weight chitosan in a finely disintegrated amorphous form. The three other drugs showed the X-ray diffraction patterns completely identical with that of the physical mixture also in the solid dispersion and no decrease in the crystallinity of the drug by the admixing of the low molecular weight chitosan.

Example 6

Flurbiprofen and indomethacin which have each a melting point lower than that of the low molecular weight chitosan were examined by differential thermal analysis. The examinations were made for the physical mixtures and solid dispersions as obtained in Example 5 and the low molecular weight chitosan alone as well as each drug without any additive. The differential thermal analysis was carried out on a differential thermal analyzer Thermoflex TG-8110 of Rigakudenki K.K. using each such an amount of the sample that corresponds to 5 mg of the drug as calculated and employing $\alpha$ -alumina as the reference substance at a heat elevation rate of 10 °C /min. The results are recitded in Figs. 7(A) and 7(B).

By differential thermal analysis, physical state of the drug in the solid can be conjectured by observing endothermic peaks over the temperature axis on the chart occurring by possible melting of the drug, as seen from the charts of Fig. 7, which show that the endothermic peak due to melting of the drug has disappeared for the solid dispersion of flurbiprofen, indicating that flurbiprofen is present therein in a state finely dispersed within the mass of said solid dispersion losing its crystalline structure. In the case of indomethacin, endothermic peak was found also for the solid dispersion, indicating that indomethacin is present therein under dispersion within the mass under preservation of its crystalline structure. These results corresponds to those of Example 5 and it is to be assumed that flurbiprofen is held dispersed within the solid dispersion thereof together with the low molecular weight chitosan in an amorphous state under some interaction with the low molecular weight chitosan in the solid state, while the other drugs cause almost no interaction with the low molecular weight chitosan and will be dispersed within the solid dispersion holding their crystalline structure.

Example 7

Flurbiprofen, indomethacin and triamcinolone were examined for evaluating wetting characteristics with water. Examination was effected for the samples of the solid dispersions as obtained in Example 5 and for each drug alone without any additive. The wetting was evaluated by determining the wetting contact angle of water drop on the surface of a tablet having a diameter of 2 cm obtained using an IR tabletting machine with a drop of water of 50 μl. The results are summarized in Table 4.

Table 4

| Drug | Wetting Contact Angle (° ) | |
|---|---|---|
| | Drug alone | Solid Disp. |
| Flurbiprofen | 55 | 30 |
| Indomethacin | 50 | 32 |
| Triamcinolone | 50 | 40 |

As seen from Table 4, the wetting contact angle for the solid dispersion with the low molecular weight chitosan was found to be decreased as compared with that for the drug alone. The results suggest that the low molecular weight chitosan contributes to the increase in the dissolution rate of the poorly water-soluble drugs by improving the wettability by water on the surface of the drug particle.

Example 8

Suppositories were prepared using H-15 (cacao butter) as the base substance from each of kneaded mixtures of flurbiprofen with either of the low molecular weight chitosan products C-2, C-4 and C-5 as obtained in Reference Example 1 in a weight ratio of 1 : 1 or 1 : 5. Using these suppositories, liberation rate of flurbiprofen from the suppository was detected. The results are given in Fig. 8 as a graph.

POSSIBILITIES OF APPLICATION IN INDUSTRY

The drug compositions according to the present invention can be utilized for improving the solubility and dissolution rate of various poorly water-soluble drugs for medical and other uses and can be employed as drug compositions for preparing medicinal drugs and other drugs in which the bioabsorptivity and the biouptake of such poorly water-soluble drugs in living organisms.

## Claims

1. A drug composition containing one or more drugs being poorly water soluble and thereby having poor or insufficient bioavailability and a water-soluble chitosan having an average molecular weight of between 500 and 50 x $10^3$.

2. A drug composition as claimed in Claim 1, in which the chitosan has an average molecular weight in the range from 800 to 10 x $10^3$.

3. A drug composition as claimed in Claim 1 or Claim 2, in which the chitosan is present in an amount in the range from 0.2 to 10 parts by weight per one part by weight of the drug.

4. A drug composition as claimed in any of Claims 1 to 3, wherein the drug is selected from betamethasone, diclofenac acid, dicumarol, digoxin, flurbiprofen, ibuprofen, indomethacin, ketoprofen, mefenamic acid, phenytoin, prednisolone, triamcinolone, vitamin E and vitamin K.

## Patentansprüche

1. Medikamentzusammensetzung mit einem oder mehreren Medikamenten, die schlecht wasserlöslich sind und dadurch eine schlechte oder ungenügende Bioverfügbarkeit haben, und einem wasserlöslichen Chitosan, das ein durchschnittliches Molekulargewicht von zwischen 500 und 50 x $10^3$ hat.

2. Medikamentzusammensetzung nach Anspruch 1, dadurch gekennzeichnet, daß das Chitosan ein durchschnittliches Molekulargewicht im Bereich von 800 bis 10 x $10^3$ hat.

3. Medikamentzusammensetzung nach Anspruch 1 oder 2, dadurch gekennzeichnet, daß das Chitosan in einem Mengenbereich von 0,2 bis 10 Gewichtsteilen pro Gewichtseinheit des Medikamentes vorhanden ist.

4. Medikamentzusammensetzung nach einem der Ansprüche 1 bis 3, dadurch gekennzeichnet, daß das Medikament aus Betamethason, Diclofenacsäure, Dicumarol, Digoxin, Flurbiprofen, Ibuprofen, Indomethacin, Ketoprofen, Mefenamicsäure, Phenytoin, Prednisolon, Triamcinolon, Vitamin E und Vitamin K ausgewählt ist.

## Revendications

1. Composition de médicament contenant un ou plusieurs médicaments qui sont peu solubles dans l'eau et dont la biodisponibilité est médiocre pour cette raison, et un chitosane soluble dans l'eau ayant une masse moléculaire moyenne comprise entre 500 et 50 x $10^3$.

2. Composition de médicament selon la revendication 1, dans laquelle le chitosane a une masse moléculaire moyenne dans l'intervalle compris entre 800 et 10 x $10^3$.

3. Composition de médicament selon la revendication 1 ou 2, dans laquelle le chitosane est présent dans une quantité située dans l'intervalle de 0,2 à 10 parties en poids pour une partie en poids du médicament.

4. Composition de médicament selon l'une quelconque des revendications 1 à 3, dans laquelle le médicament est choisi parmi la bêtaméthasone, le diclofenac acide, le dicumarol, la digoxine, le flurbiprofène, l'ibuprofène, l'indométhacine, le cétoprofène, l'acide méfénamique, le phénytoïne, la prednisolone, la triamcinolone, la vitamine E et la vitamine K.

Fig. 1

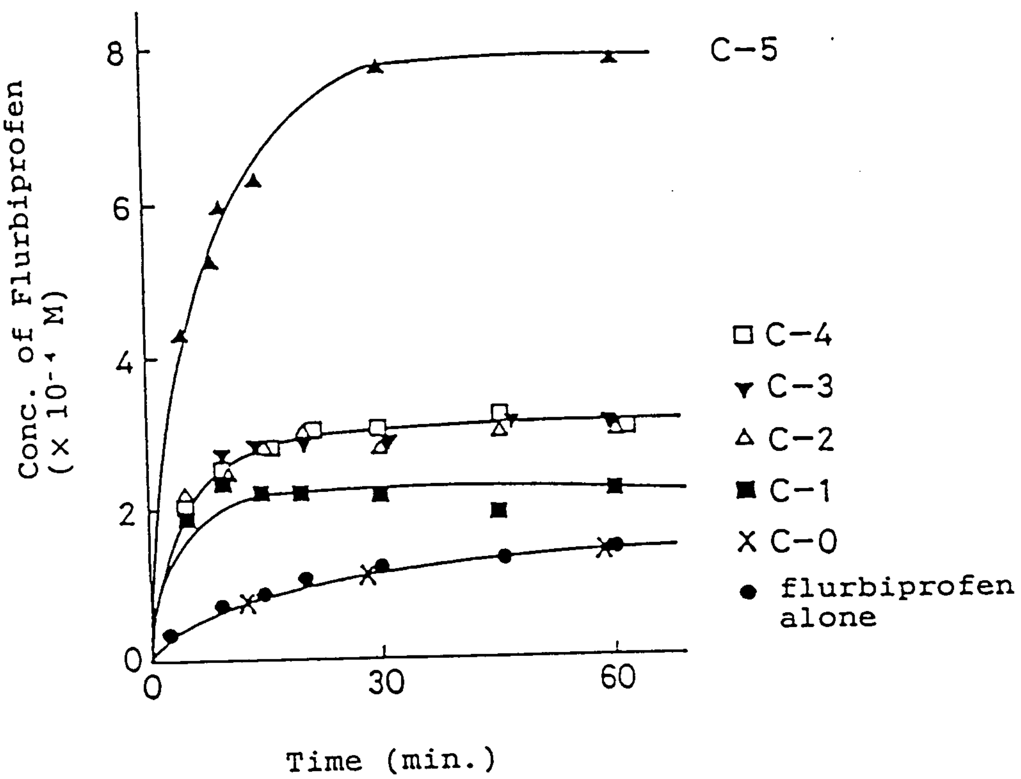
Conc. of Flurbiprofen
(× 10⁻⁴ M)
8
6
4
2
0
0
30
60
Time (min.)
C-5
C-4
C-3
C-2
C-1
C-0
flurbiprofen alone

Fig. 2

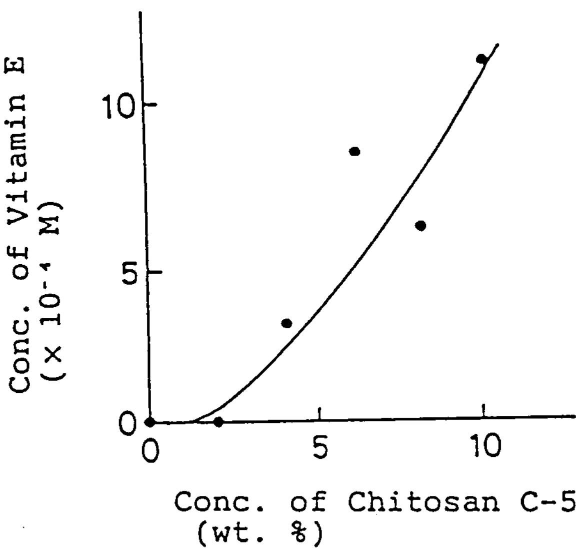
Conc. of Vitamin E
(× 10⁻⁴ M)
10
5
0
0
5
10
Conc. of Chitosan C-5
(wt. %)

Fig. 3

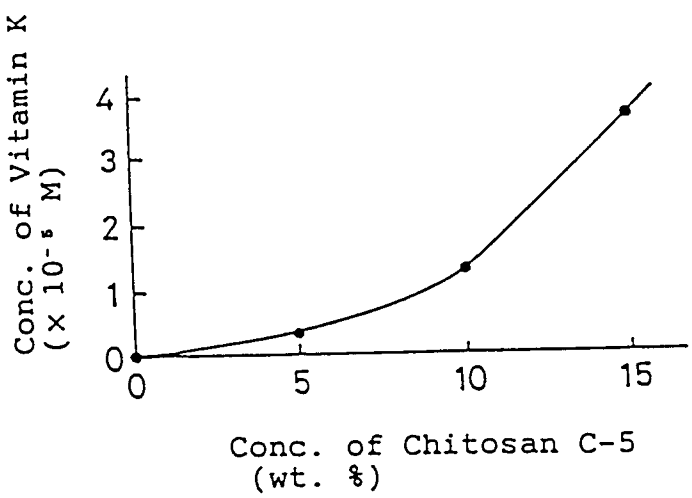
Conc. of Vitamin K
(× 10⁻⁵ M)
4
3
2
1
0
0
5
10
15
Conc. of Chitosan C-5
(wt. %)

# F i g . 4

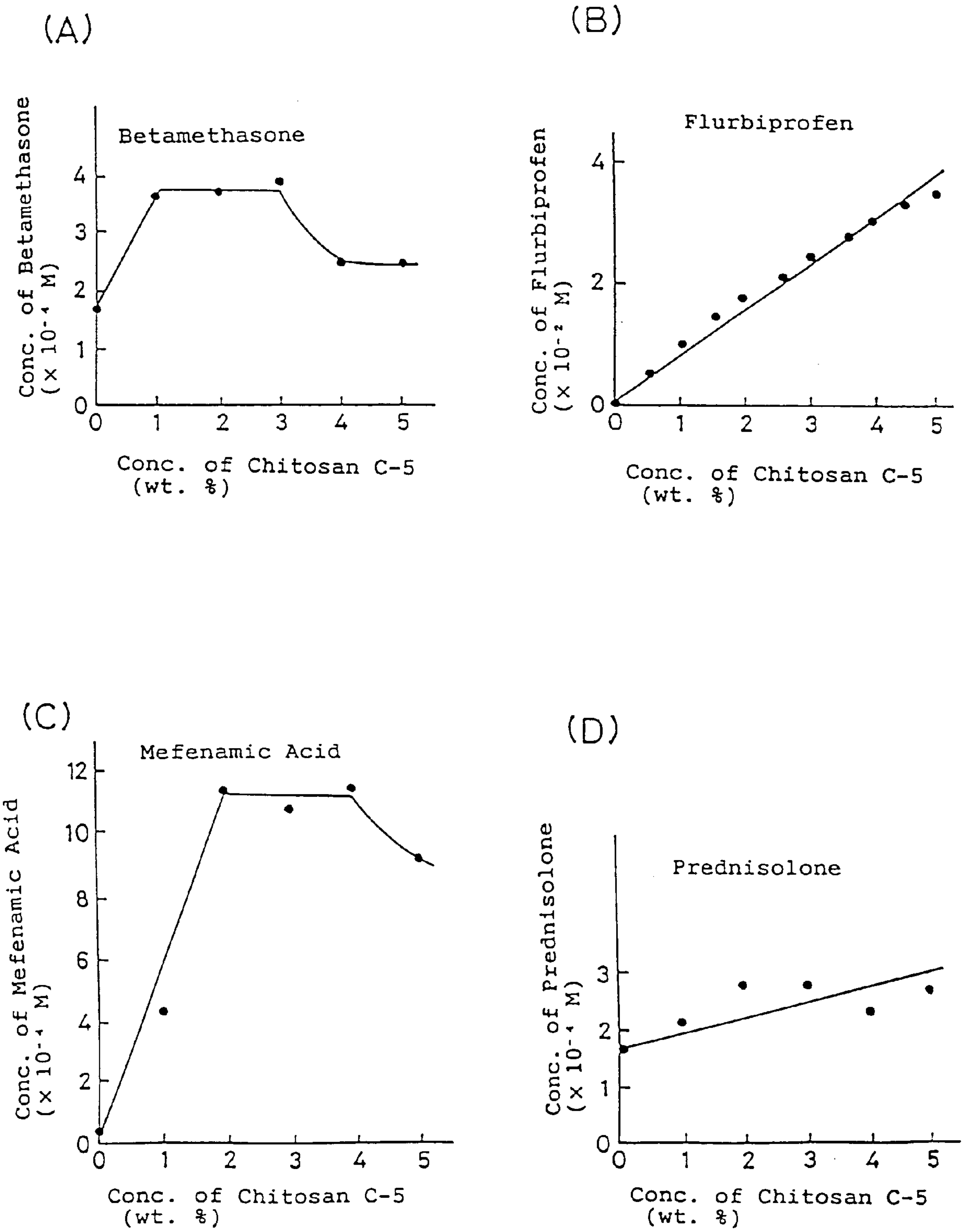
(A)
Betamethasone
Conc. of Betamethasone (× 10⁻⁴ M)
Conc. of Chitosan C-5 (wt. %)
(B)
Flurbiprofen
Conc. of Flurbiprofen (× 10⁻² M)
Conc. of Chitosan C-5 (wt. %)
(C)
Mefenamic Acid
Conc. of Mefenamic Acid (× 10⁻⁴ M)
Conc. of Chitosan C-5 (wt. %)
(D)
Prednisolone
Conc. of Prednisolone (× 10⁻⁴ M)
Conc. of Chitosan C-5 (wt. %)

Fig. 4

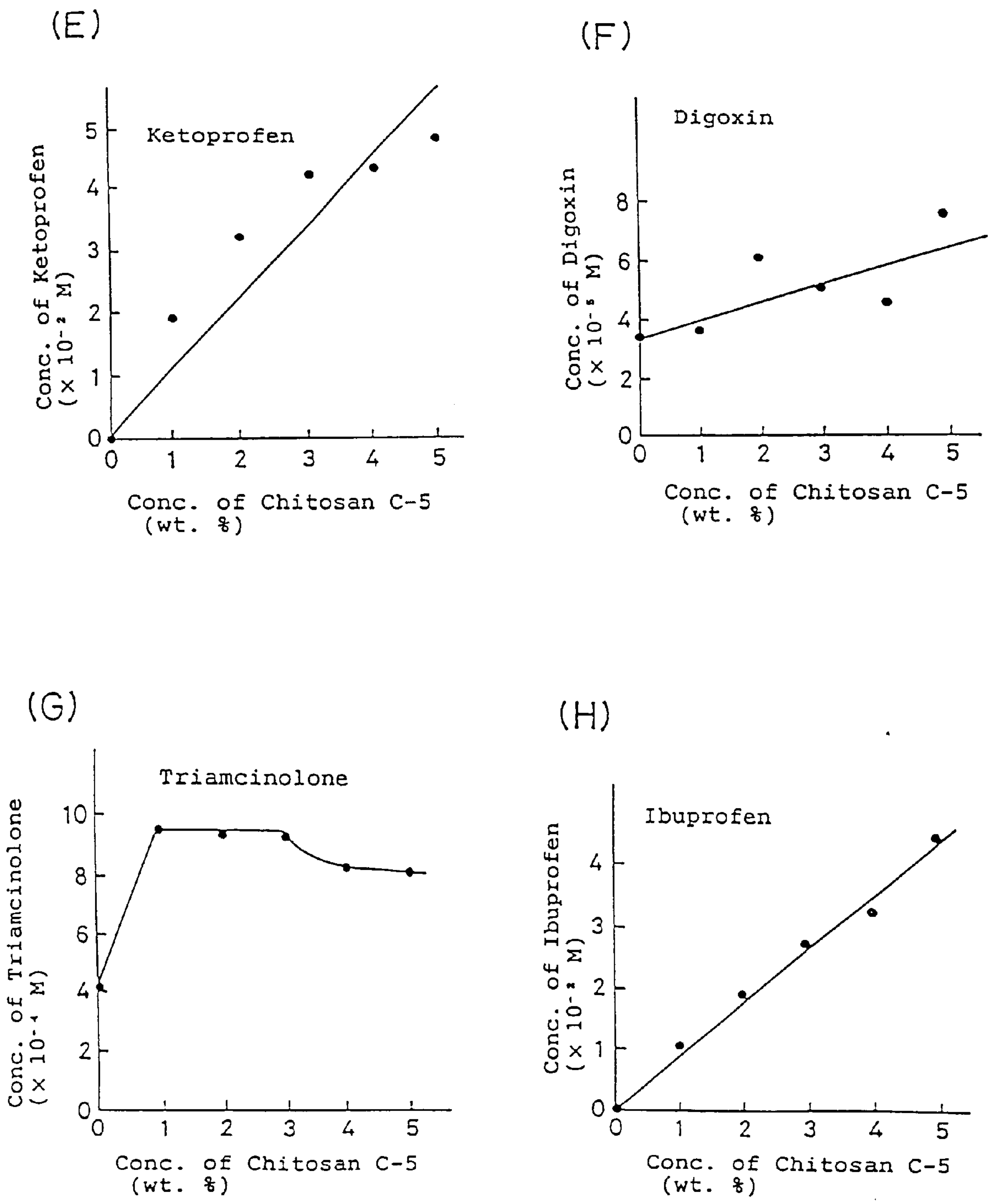
(E)
Ketoprofen
Conc. of Ketoprofen (× 10⁻² M)
Conc. of Chitosan C-5 (wt. %)
(F)
Digoxin
Conc. of Digoxin (× 10⁻⁵ M)
Conc. of Chitosan C-5 (wt. %)
(G)
Triamcinolone
Conc. of Triamcinolone (× 10⁻⁴ M)
Conc. of Chitosan C-5 (wt. %)
(H)
Ibuprofen
Conc. of Ibuprofen (× 10⁻² M)
Conc. of Chitosan C-5 (wt. %)

Fig. 4

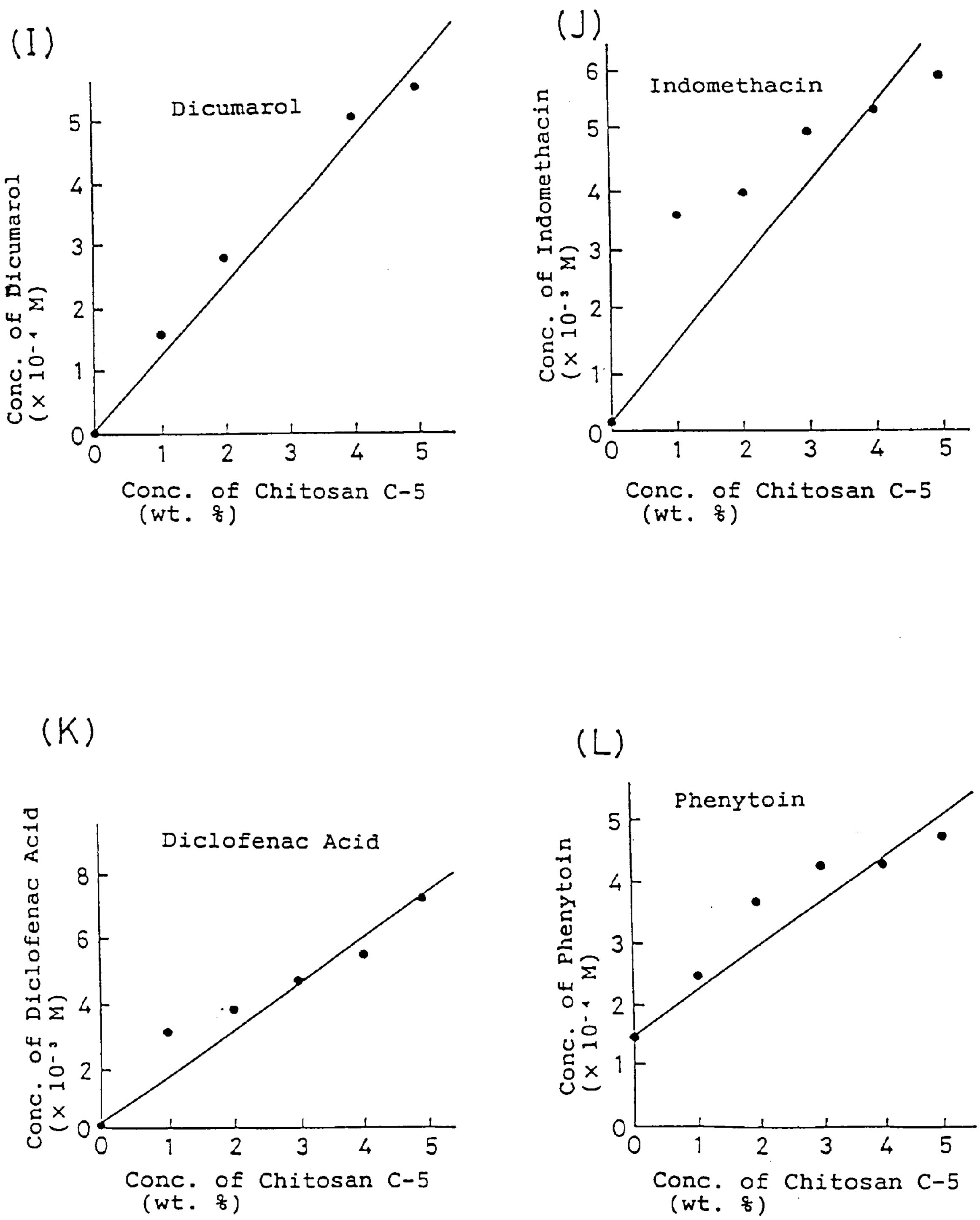
(I)
Dicumarol
Conc. of Dicumarol (× 10⁻⁴ M)
Conc. of Chitosan C-5 (wt. %)
(J)
Indomethacin
Conc. of Indomethacin (× 10⁻³ M)
Conc. of Chitosan C-5 (wt. %)
(K)
Diclofenac Acid
Conc. of Diclofenac Acid (× 10⁻³ M)
Conc. of Chitosan C-5 (wt. %)
(L)
Phenytoin
Conc. of Phenytoin (× 10⁻⁴ M)
Conc. of Chitosan C-5 (wt. %)

# Fig. 5

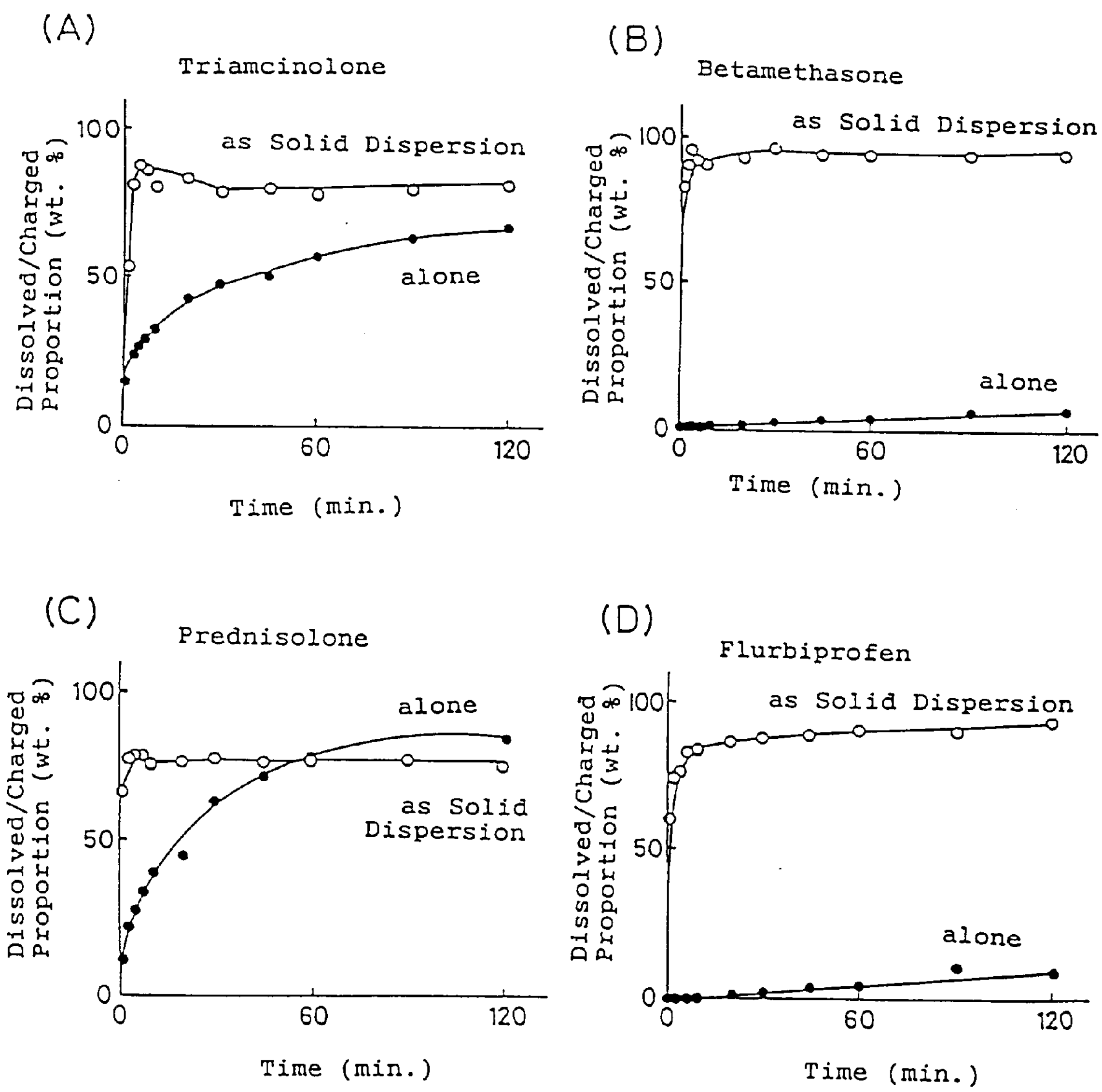
(A)
Triamcinolone
as Solid Dispersion
alone
Dissolved/Charged Proportion (wt. %)
100
50
0
0
60
120
Time (min.)
(B)
Betamethasone
as Solid Dispersion
alone
Dissolved/Charged Proportion (wt. %)
100
50
0
0
60
120
Time (min.)
(C)
Prednisolone
alone
as Solid Dispersion
Dissolved/Charged Proportion (wt. %)
100
50
0
0
60
120
Time (min.)
(D)
Flurbiprofen
as Solid Dispersion
alone
Dissolved/Charged Proportion (wt. %)
100
50
0
0
60
120
Time (min.)

F i g . 5

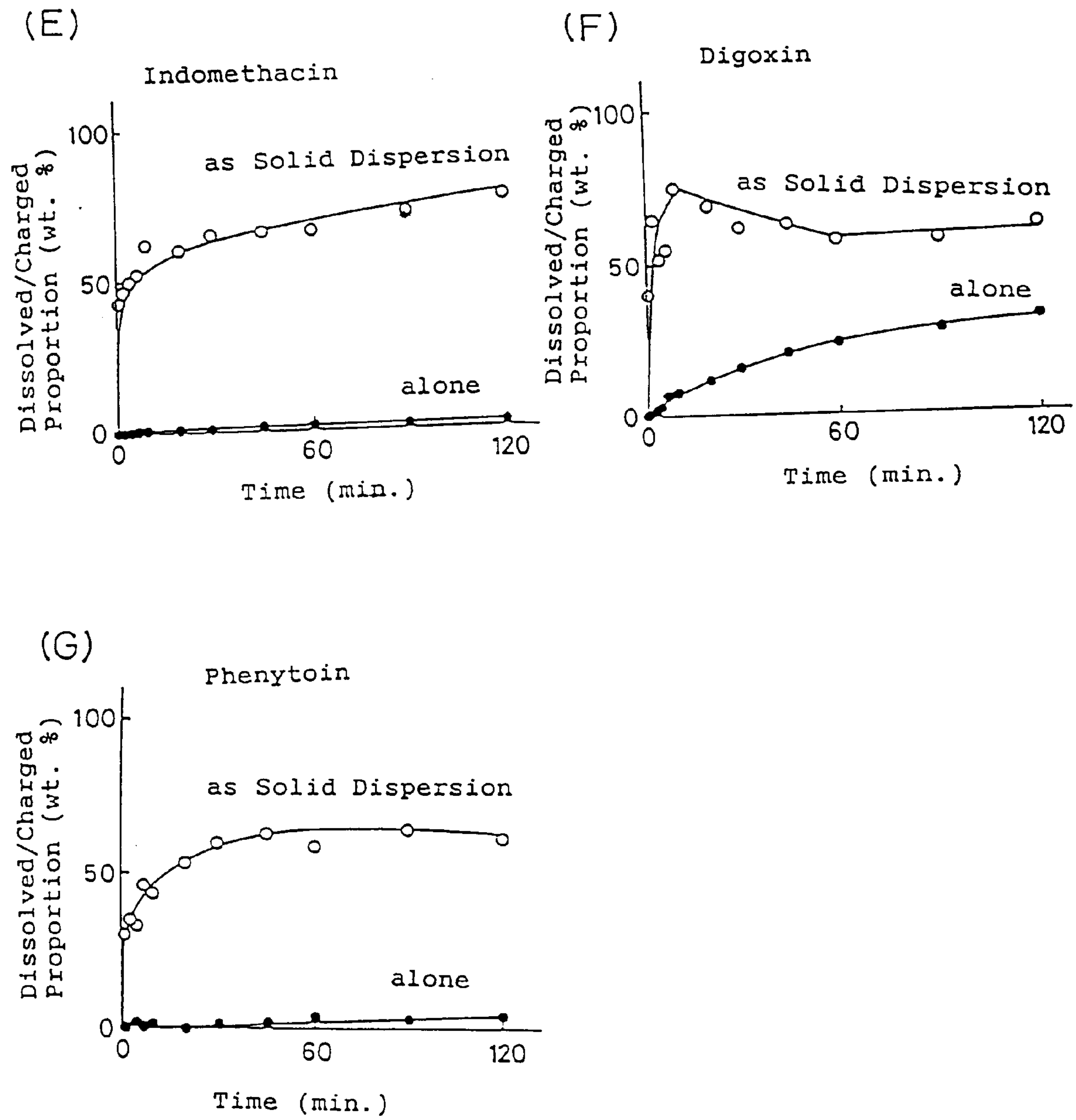
(E)
Indomethacin
as Solid Dispersion
alone
Dissolved/Charged Proportion (wt. %)
100
50
0
0
60
120
Time (min.)
(F)
Digoxin
as Solid Dispersion
alone
Dissolved/Charged Proportion (wt. %)
100
50
0
0
60
120
Time (min.)
(G)
Phenytoin
as Solid Dispersion
alone
Dissolved/Charged Proportion (wt. %)
100
50
0
0
60
120
Time (min.)

Fig. 6

(A) Flurbiprofen

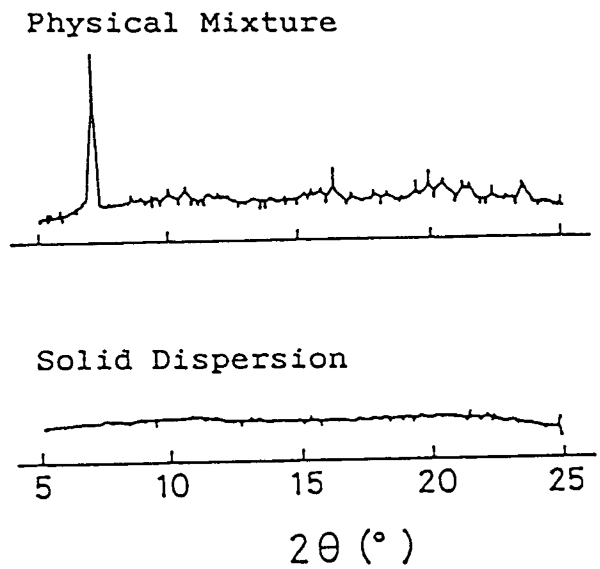


(B) Indomethacin

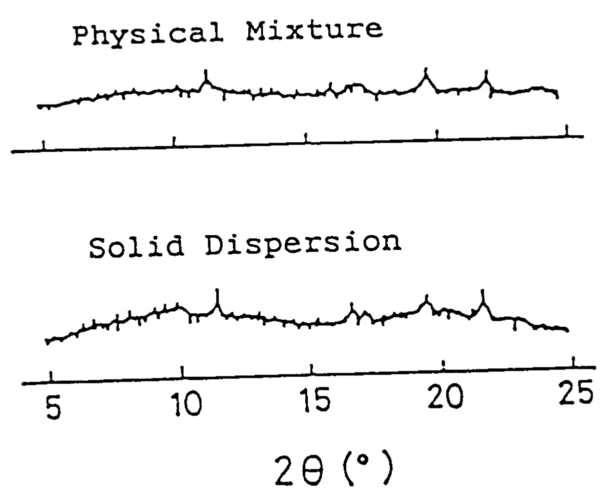

# F i g . 6

(C) Digoxin

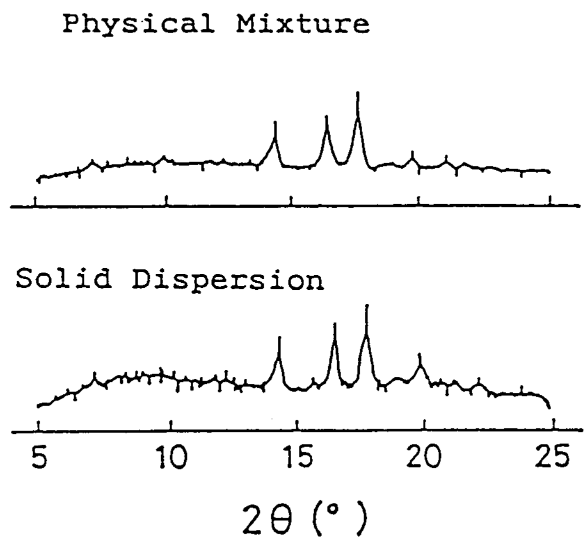


(D) Triamcinolone

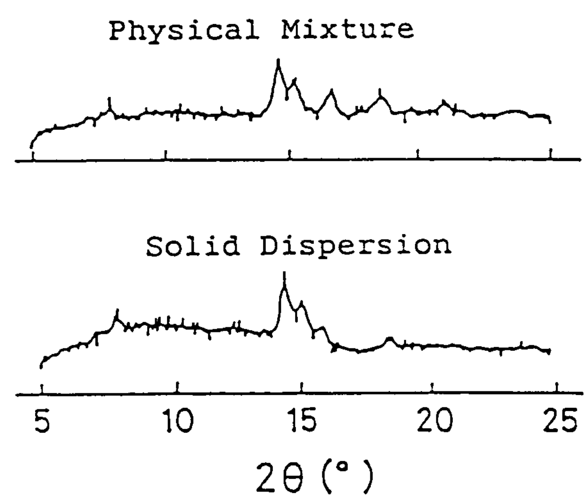

Fig. 7

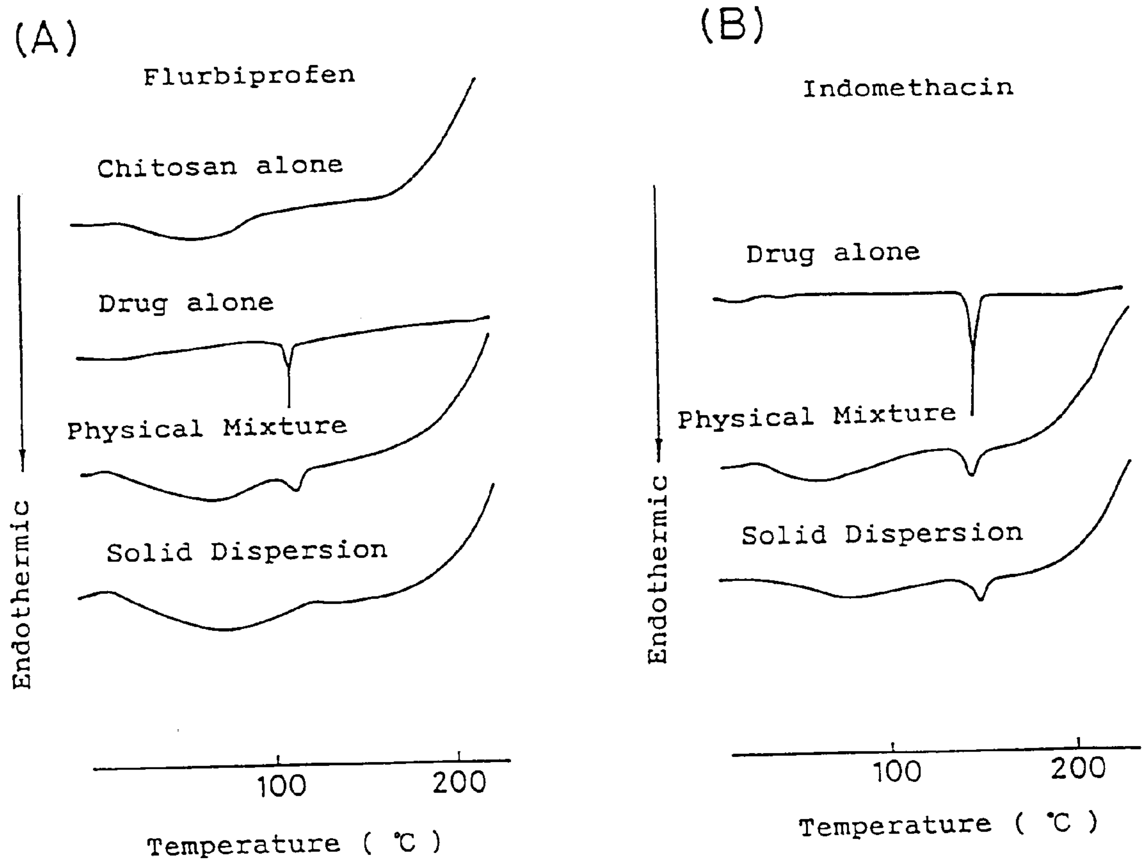
(A)
Flurbiprofen
Chitosan alone
Drug alone
Physical Mixture
Solid Dispersion
Endothermic
100
200
Temperature ( ℃ )
(B)
Indomethacin
Drug alone
Physical Mixture
Solid Dispersion
Endothermic
100
200
Temperature ( ℃ )

F i g . 8

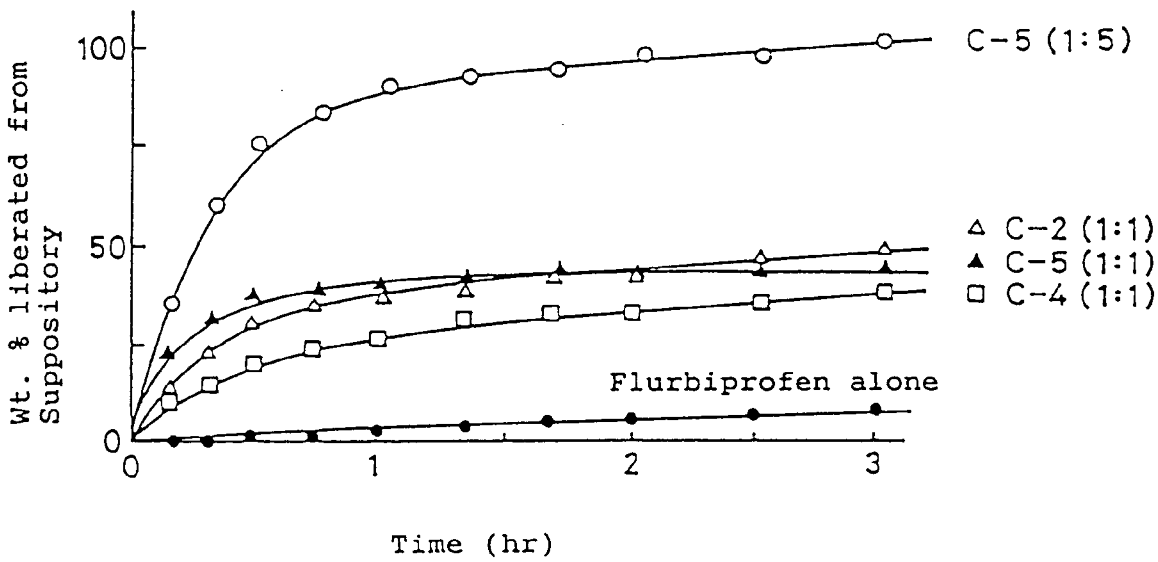
100
50
0
0
1
2
3
Wt. % liberated from Suppository
Time (hr)
C-5 (1:5)
△ C-2 (1:1)
▲ C-5 (1:1)
□ C-4 (1:1)
Flurbiprofen alone